# EUROPEAN PATENT SPECIFICATION

(11) **EP 2 468 361 B1**
(45) Date of publication and mention of the grant of the patent: **21.08.2013**
(21) Application number: 11194770.1
(22) Date of filing: 21.12.2011
(51) Int. Cl.: A61P 3/10

(54) **Vildagliptin Formulations**
Vildaglitptin-Formulierungen
Formulations de vildagliptine

(30) Priority: 21.12.2010 TR 201010683
(43) Date of publication of application: 27.06.2012
(73) Proprietor: Sanovel Ilaç Sanayi Ve Ticaret Anonim Sirketi, 34460 Sariyer/Istanbul (TR)
(72) Inventor: Cifter, Ümit, 34398 Istanbul (TR); Türkyilmaz, Ali, 34398 Istanbul (TR); Saydam, Mehtap, 34398 Istanbul (TR); Ergenekon, Ercan, 34398 Istanbul (TR)
(74) Representative: Sevinç, Erkan

(56) References cited:
- WO-A1-2006/047248
- WO-A2-2007/041053
- WO-A2-2008/146178
- WO-A2-2009/121945
- US-A1- 2007 172 525
- US-A1- 2010 074 950

## Description

### Field of Invention

The present invention relates to formulations of vildagliptin or a pharmaceutically acceptable salt of vildagliptin. The present invention particularly relates to stable tablet or capsule formulations of vildagliptin with desired levels of solubility and dissolution rate.

### Background of Invention

Vildagliptin is a dipeptidyl dipeptidase-IV (DPP-IV) inhibitor developed for use in the treatment of type 2 diabetes (non-insulin dependent diabetes). Vildagliptin inhibits the degradation of the dipeptidil dipeptidase-IV enzyme, thereby inhibiting the effects of incretin hormones, glucagon-like peptide-1 (GLP-1), and of glucose-dependent insulinotropic peptide (GIP). The chemical designation of vildagliptin is (S)-{[(3-hydroxyadamantan-1-yl)amino]acetyl}pyrrolidine-2-carbonitril, with the chemical structure illustrated below in Formula 1.

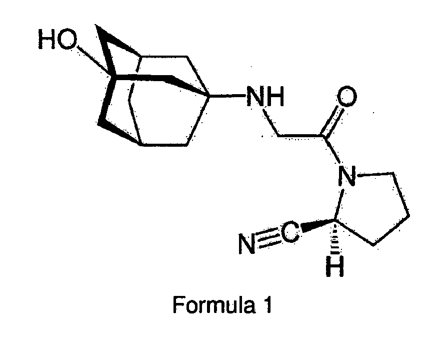

Vildagliptin is soluble in water and in organic polar solvents.

Vildagliptin is marketed under the trademark Galvus^{®} in 50 mg dosage forms. It is used against diabetes mellitus, but particularly in treating type 2 diabetes.

There have been various patents available in the patent literature in relation to vildagliptin.

The patent application WO00/34241 discloses vildagliptin or an acid addition salt thereof, as well as their use in diabetes mellitus and obesity.

The patent application WO2006/078593 claims a direct-compression formulation of a DPP-IV inhibitor compound, and preferably of vildagliptin or an acid addition salt thereof.

The patent application WO2006/135723 discloses a formulation, comprising vildagliptin as an active agent, as well as hydroxypropyl methylcellulose, microcrystalline cellulose, and magnesium stearate.

Stability-related problems do occur in a many active agents, including vildagliptin, under the influence of ambient and physical conditions. Vildagliptin is an active agent that is highly-susceptible to air and humidity. When vildagliptin is exposed to air and humidity, it degrades structurally and develops behavioral changes. The stability of vildagliptin products developed is not of desired level and the shelf life thereof is shortened. In addition, vildagliptin is reactive against the excipients employed in developing formulations containing the same. This fact causes impurities to occur in the formulation and leads to incorporation of undesired components into the formulation.

Based on the drawbacks mentioned above, a novelty is necessitated in the art of formulations of vildagliptin used in treating diabetes mellitus.

### Objects and Brief Description of Invention

The present invention provides a vildagliptin formulation, eliminating all aforesaid problems and bringing additional advantages to the relevant prior art.

Accordingly, the main object of the present invention is to obtain a formulation of vildagliptin, which is stable and has desired levels of solubility and dissolution rate.

Another object of the present invention is to develop a coating, providing stability for vildagliptin-containing formulations and not affecting the solubility and dissolution rates thereof.

A further object of the present invention is to obtain a stable vildagliptin formulation with high bioavailability.

Another object of the present invention is to provide a high dissolution rate for such stable vildagliptin formulations having high bioavailability.

A pharmaceutical formulation is realized to carry out all objects, referred to above and to emerge from the following detailed description.

According to a preferred embodiment of the present invention, said novelty is embodied with vildagliptin granules having at least one coating layer and with one or more excipient.

According to a preferred embodiment of the present invention, the weight ratio of vildagliptin to the coating layers is in the range of 3 to 100, preferably in the range of 5 to 50, and more preferably in the range of 8 to 20.

According to a preferred embodiment of the present invention, the coating layer comprises pullulan or polyvinyl alcohol or a properly-proportioned mixture thereof.

According to a preferred embodiment of the present invention, said formulation is in the form of a capsule, a tablet, powder, granules or a sachet.

According to a preferred embodiment of the present invention, said excipient contains at least one or a properly-proportioned mixture of fillers, binders, disintegrants, lubricants, and glidants.

According to a preferred embodiment of the present invention, said filler comprises at least one or a mixture of microcrystalline cellulose, pullulan, dextrin, mannitol, sorbitol, lactose, dicalcium phosphate, calcium phosphate dibasic anhydrous, with the preferred filler being microcrystalline cellulose or pullulan, or a properly-proportioned mixture thereof.

According to a preferred embodiment of the present invention, said binder comprises at least one or a mixture of polyvinylpyrrolidone (povidone), hydroxypropyl methylcellulose, hydroxypropyl cellulose, hydroxyethyl cellulose, pregelatinized starch, sodium alginate, chitosane, carboxymethyl cellulose, methyl cellulose, ethyl cellulose, and other cellulose derivatives, with the preferred binder being polyvinylpyrrolidone.

According to a preferred embodiment of the present invention, said glidant comprises at least one or a mixture of colloidal silicone dioxide, talk, aluminum silicate, tribasic calcium phosphate, magnesium silicate, with the preferred glidant being colloidal silicone dioxide.

According to a preferred embodiment of the present invention, said disintegrant comprises at least one or a properly-proportioned mixture of sodium starch glycolate, croscarmellose sodium, crospovidone, sodium alginate, gums, starch, and magnesium aluminum silicate, with the preferred disintegrant being croscarmellose sodium.

According to a preferred embodiment of the present invention, suitable lubricant comprises at least one or a properly-proportioned mixture of sodium stearyl fumarate, magnesium stearate, polyethylene glycol, stearic acid, metal stearates, boric acid, sodium chloride benzoate and acetate, sodium or magnesium lauryl sulfate, with the preferred lubricant being magnesium stearate.

A further preferred embodiment according to the present invention provides a method for preparing a solid pharmaceutical formulation, this method comprising the steps of
a. coating vildagliptin granules with pullulan or polyvinyl alcohol, or with a properly-proportioned mixture thereof,
b. mixing the coated granules obtained with filling, binding, and disintegrating agents,
c. adding magnesium stearate into the mixture obtained, and continuing to mix it,
d. compressing the mixture into tablets, or filling into capsules,
e. film-coating the tablets compressed.

In a further preferred embodiment according to the present invention, said pharmaceutical formulation consisting of:
a. core
   a. vildagliptin or a pharmaceutically acceptable salt or polymorph thereof at 3.5 to 90% by weight,
   b. pullulan at 0.2 to 10.0 % by weight,
   c. microcrystalline cellulose at 2.0 to 90.0 % by weight,
   d. polyvinylpyrrolidone at 0.25 to 10.0 % by weight,
   e. croscarmellose sodium at 0.2 to 20.0 % by weight,
   f. colloidal silicone dioxide at 0.1 to 5.0 % by weight,
   g. magnesium stearate at 0.1-5.0 % by weight,
b. granule coating
   a. polyvinyl alcohol at 0.5 to 5.0 % by weight, or
   b. pullulan or a properly-proportioned mixture thereof at 0.5 to 5.0 % by weight.

### Detailed Description of Invention

### Example 1:

| Unit Formula | Amount in tablet (%) |
|---|---|
| core tablet | |
| vildagliptin | 30,0 |
| pullulan | 2,0 |
| microcrystalline cellulose | 52,5 |
| polyvinylpyrrolidone | 2,0 |
| croscarmellose sodium | 8,0 |
| colloidal silicone dioxide | 0,5 |
| magnesium stearate | 1,0 |
| film coating | |
| Opadry AMB -OY- B 28920 white | 4,0 |

### Example 2:

| Unit Formula | Amount in tablet (%) |
|---|---|
| core tablet | |
| vildagliptin | 89,0 |
| polyvinyl alcohol | 5,0 |
| magnesium stearate | 1,0 |
| film coating | |
| Opadry AMB -OY- B 28920 white | 5,0 |

### Example 3:

| Unit Formula | Amount in tablet (%) |
|---|---|
| core tablet | |
| vildagliptin | 89,0 |
| pullulan | 5,0 |
| magnesium stearate | 1,0 |
| film coating | |
| Opadry AMB -OY- B 28920 white | 5,0 |

### Example 4:

| Unit Formula | Amount in tablet (%) |
|---|---|
| core tablet | |
| vildagliptin | 89,0 |
| polyvinyl alcohol | 3,0 |
| pullulan | 2,0 |
| magnesium stearate | 1,0 |
| film coating | |
| Opadry AMB -OY- B 28920 white | 5,0 |

The formulation according to the present invention is prepared as follows. In the embodiment according to Example 1, vildagliptin is coated in a fluidized bed with pullulan and is sieved thereafter. The coated granules are mixed with filling, binding, and disintegrating agent. Into the mixture obtained is added magnesium stearat. The final mixture is compressed into tablets and coated, or filled into capsules.

In the embodiments according to examples 2 and 3, vildagliptin is subjected to a wet granulation process with polyvinyl alcohol or pullulan and is then dried in a fluidized bed, or is subjected to a dry granulation process. It is then dried. Other excipients are added. Then, it is compressed into tablets and coated. Similarly in examples 2 and 3, vildagliptin is coated with polyvinyl alcohol or pullulan in a fluidized bed or is subjected to wet granulation with alcohol and is dried in a fluidized bed. It is then dried. It is filled into HPMC capsules. In a different embodiment according to examples 2 and 3, vildagliptin is subjected to dry granulation with polyvinyl alcohol or pullulan. It is then dried. It is filled into HPMC capsules. In Example 4, all processes performed for examples 2 and 3 are now done by using a mixture of pullulan + PVA (Polyvinyl alcohol). The tablets or capsules are packaged in ALU-ALU blister.

With the invention realized, stable vildagliptin formulations can be obtained which have surprisingly good solubility and dissolution rates, and thus high bioavailability. Particularly the ingredients pullulan and/or polyvinyl alcohol used in coating the active agent has/have surprisingly increased the product stability and prolonged its shelf life.

Additionally, applying a coating comprising hydroxypropyl cellulose and hydroxypropyl methylcellulose contributes in improving the formulation stability.

The active agent vildagliptin used in said formulation can be preferred in the form of a pharmaceutically acceptable salt, polymorph, enantiomer thereof.

The formulation obtained is used in preventing and treating diabetes mellitus. The formulation obtained is particularly used in treating type 2 diabetes mellitus.

The term granule, as used and defined herein, means powder, particle, granule, or pellet forms of vildagliptin or of a pharmaceutically acceptable salt of vildagliptin.

It is further possible to use the following additional excipients in the formulation.

Suitable coating agents include, but are not restricted to hydroxypropyl methylcellulose, polyethylene glycol, polyvinylpyrrolidone, polyvinylpyrrolidone-vinyl acetate copolymer (PVP-VA), pullulan like polymers, and all kinds of Opadry, as well as pigments, dyes, titanium dioxide and iron oxide, talk.

Suitable colorants include, but are not restricted to at least one or a mixture of food, drug, and cosmetic (FD&C) dyes (e.g. FD&C blue, FD&C green, FD&C red, FD&C yellow, FD&C lake), ponceau, indigo drug & cosmetic (D&C) blue, indigotine FD&C blue, carmoisine indigotine (indigo Carmine); iron oxides (e.g. iron oxide red, yellow, black), quinoline yellow, flame red, brilliant red (carmine), carmoisine, sunset yellow.

Suitable preservatives include, but are not restricted to at least one or a mixture of methylparaben and propylparaben and the salts thereof (e.g. sodium or potassium salts), sodium benzoate, citric acid, benzoic acid, butylated hydroxytoluene and butylated hydroxyanisole, etc..

The protection scope of the present invention is set forth in the annexed claims and cannot be restricted to the illustrative disclosures given above, under the detailed description. Any alternative embodiments to be produced by persons skilled in the art according to the basic principles, which are under the protection scope as set forth in the claims, shall be an infringement of the present invention.

## Claims

1. A pharmaceutical formulation, comprising
a. vildagliptin granules which are coated with at least one coating layer comprising pullulan or polyvinyl alcohol, or a mixture thereof and,
b. one or more excipients.

2. The pharmaceutical formulation according to Claim 1, wherein the weight ratio of vildagliptin to the coating layer is in the range of 3 to 100, preferably of 5 to 50, and more preferably of 8 to 20.

3. The pharmaceutical formulation according to any of the preceding claims, this formulation being in the form of a capsule, tablet, powder, granule, or sachet.

4. The pharmaceutical formulation according to any of the preceding claims, wherein said excipient comprises at least one or a mixture of fillers, binders, disintegrants, lubricants, and glidants.

5. The pharmaceutical formulation according to any of the preceding claims, further comprising, as a filling agent, at least one or a mixture of microcrystalline cellulose, pullulan, dextrin, mannitol, sorbitol, lactose, dicalcium phosphate, calcium phosphate dibasic anhydrous, with the preferred filling agent being microcrystalline cellulose or pullulan, or a properly-proportioned mixture thereof.

6. The pharmaceutical formulation according to any of the preceding claims, wherein said binding agent comprises at least one or a properly-proportioned mixture of polyvinylpyrrolidone (povidone), hydroxypropyl methylcellulose, hydroxypropyl cellulose, hydroxyethyl cellulose, pregelatinized starch, sodium alginate, chitosane, carboxymethyl cellulose, methyl cellulose, ethyl cellulose, and other cellulose derivatives, with the preferred binding agent being polyvinylpyrrolidone.

7. The pharmaceutical formulation according to any of the preceding claims, wherein said glidant comprises at least one or a properly-proportioned mixture of colloidal silicone dioxide, talk, aluminum silicate, tribasic calcium phosphate, magnesium silicate, with the preferred glidant being colloidal silicone dioxide.

8. The pharmaceutical formulation according to any of the preceding claims, wherein said disintegrant comprises at least one or a properly-proportioned mixture of sodium starch glycolate, croscarmellose sodium, crospovidone, sodium alginate, gums, starch, and magnesium aluminum silicate, with the preferred disintegrant being croscarmellose sodium.

9. The pharmaceutical formulation according to any of the preceding claims, wherein said lubricant comprises at least one or a properly-proportioned mixture of sodium stearyl fumarate, magnesium stearate, polyethylene glycol, stearic acid, metal stearates, boric acid, sodium chloride benzoate and acetate, sodium or magnesium lauryl sulfate, with the preferred lubricant being magnesium stearate.

10. A method for preparing a pharmaceutical formulation according to any of the preceding claims, comprising the steps of:
a. coating vildagliptin granules with pullulan or polyvinyl alcohol, or with a properly-proportioned mixture thereof,
b. mixing the coated granules obtained with filling, binding, and disintegrating agents,
c. adding magnesium stearate into the mixture obtained, and continuing to mix it,
d. compressing the mixture into tablets, or filling it into capsules,
e. film-coating the tablets compressed.

11. The pharmaceutical formulation according to any of the preceding claims, consisting of:
a. core comprising the following ingredients;
a. vildagliptin or a pharmaceutically acceptable salt or polymorph thereof at 3.5 to 90% by weight,
b. pullulan at 0.2 to 10% by weight,
c. microcrystalline cellulose at 2.0 to 90.0 % by weight,
d. polyvinylpyrrolidone at 0.25 to 10.0 % by weight,
e. croscarmellose sodium at 0.2 to 20.0 % by weight,
f. colloidal silicone dioxide at 0.1 to 5.0 % by weight,
g. magnesium stearate at 0.1-5.0 % by weight,
b. granule coating comprising the following ingredients;
a. polyvinyl alcohol at 0.5 to 5.0 % by weight, or
b. pullulan or a mixture thereof at 0.5 to 5.0 % by weight.

12. The pharmaceutical formulation according to any of the preceding claims for preventing or treating diabetes mellitus in mammalians and particularly in humans.

## Patentansprüche

1. Pharmazeutische Formulierung, die folgendes umfasst:
a. ein Vildagliptingranulat, das mit mindestens einer Überzugsschicht umfassend Pullulan oder Polyvinylalkohol oder eine Mischung daraus beschichtet ist,
b. einen oder mehrere Hilfsstoffe.

2. Pharmazeutische Formulierung nach Anspruch 1, bei der das Gewichtsverhältnis von Vildagliptin zur Überzugsschicht im Bereich von 3 bis 100, vorzugsweise von 5 bis 50, und noch bevorzugter von 8 bis 20 liegt.

3. Pharmazeutische Formulierung nach einem der voranstehenden Ansprüche, wobei diese Formulierung in Form einer Kapsel, einer Tablette, eines Pulvers, eines Granulats oder eines Päckchens vorliegt.

4. Pharmazeutische Formulierung nach einem der voranstehenden Ansprüche, bei der der Hilfsstoff zumindest einen Füllstoff, ein Bindemittel, ein Sprengmittel, ein Gleitmittel und ein Trennmittel oder eine Mischung dieser umfasst.

5. Pharmazeutische Formulierung nach einem der voranstehenden Ansprüche, die als Hilfsstoff des Weiteren zumindest einen der nachfolgenden Stoffe oder eine Mischung daraus umfasst: mikrokristalline Cellulose, Pullulan, Dextrin, Mannitol, Sorbitol, Laktose, Dicalciumphosphat, zweibasiges wasserfreies Calciumphosphat; wobei mikrokristalline Cellulose oder Pullulan, oder eine passend proportionierte Mischung daraus, als Füllstoff bevorzugt wird.

6. Pharmazeutische Formulierung nach einem der voranstehenden Ansprüche, bei der das Bindemittel zumindest einen der nachfolgenden Stoffe oder eine Mischung daraus umfasst: Polyvinylpyrrolidon (Povidon), Hydroxypropylmethylcellulose, Hydroxypropylcellulose, Hydroxyethylcellulose, Quellstärke, Natriumalginat, Chitosan, Carboxymethylcellulose, Methylcellulose, Ethylcellulose, und andere Cellulosederivate; wobei Polyvinylpyrrolidon als Bindemittel bevorzugt wird.

7. Pharmazeutische Formulierung nach einem der voranstehenden Ansprüche, bei der das Trennmittel zumindest einen der nachfolgenden Stoffe oder eine Mischung daraus umfasst: kolloidales Silikondioxid, Talk, Aluminiumsilikat, dreibasisches Calciumphosphat, Magnesiumsilikat; wobei kolloidales Silikondioxid als Trennmittel bevorzugt wird.

8. Pharmazeutische Formulierung nach einem der voranstehenden Ansprüche, bei der das Sprengmittel zumindest einen der nachfolgenden Stoffe oder eine passend proportionierte Mischung daraus umfasst: Natriumstärkeglykolat, Croscarmellose-Natrium, Crospovidon, Natriumalginat, Gummis, Stärke und Magnesiumaluminiumsilikat; wobei Croscarmellose-Natrium als Sprengmittel bevorzugt wird.

9. Pharmazeutische Formulierung nach einem der voranstehenden Ansprüche, bei der das Gleitmittel zumindest einen der nachfolgenden Stoffe oder eine passend proportionierte Mischung daraus umfasst: Natriumstearylfumarat, Magnesiumstearat, Polyethylenglykol, Stearinsäure, Metallstearate, Borsäure, Natriumchloridbenzoat und Azetat, Natrium- oder Magnesiumlaurylsulfat; wobei Magnesiumstearat als Gleitmittel bevorzugt wird.

10. Verfahren zur Herstellung einer pharmazeutischen Formulierung nach einem der voranstehenden Ansprüche mit folgenden Schritten:
a. Beschichten eines Vildagliptingranulats mit Pullulan oder Polyvinylalkohol oder einem passend proportionierten Gemisch daraus,
b. Mischen des erhaltenen beschichteten Granulats mit Füllstoffen, Bindemitteln und Sprengmitteln,
c. Zugeben von Magnesiumstearat in die erhaltene Mischung und weiteres Mischen dieser,
d. Komprimieren der Mischung zu Tabletten, oder Füllen der Mischung in Kapseln,
e. Filmbeschichten der komprimierten Tabletten.

11. Pharmazeutische Formulierung nach einem der voranstehenden Ansprüche, bestehend aus:
a. einem Kern mit folgenden Inhaltsstoffen:
a) Vildagliptin oder ein pharmazeutisch akzeptables Salz oder Polymorph davon zu 3,5 bis 90 Gew.-%,
b) Pullulan zu 0,2 bis 10 Gew.-%,
c) mikrokristalline Cellulose zu 2,0 bis 90,0 Gew.-%,
d) Polyvinylpyrrolidon zu 0,25 bis 10,0 Gew.-%,
e) Croscarmellose-Natrium zu 0,2 bis 20,0 Gew.-%,
f) kolloidales Silikondioxid zu 0,1 bis 5,0 Gew.-%,
g) Magnesiumstearat zu 0,1 bis 5,0 Gew.-%,
b. einem Granulatüberzug mit folgenden Inhaltsstoffen:
a) Polyvinylalkohol zu 0,5 bis 5,0 Gew.-%, oder
b) Pullulan, oder ein Gemisch daraus zu 0,5 bis 5,0 Gew.-%.

12. Pharmazeutische Formulierung nach einem der voranstehenden Ansprüche zur Prophylaxe oder Therapie von Diabetes mellitus bei Säugern und insbesondere Humanpatienten.

## Revendications

1. Formulation pharmaceutique, comprenant
a. des granulés de vildagliptine qui sont enrobés avec au moins une couche d'enrobage comprenant du pullulane ou de l'alcool polyvinylique, ou un mélange de ceux-ci et
b. un ou plusieurs excipients.

2. Formulation pharmaceutique selon la revendication 1, dans laquelle le rapport en poids de la vildagliptine à la couche d'enrobage se situe dans la plage allant de 3 à 100, de préférence de 5 à 50, plus préférentiellement de 8 à 20.

3. Formulation pharmaceutique selon l'une quelconque des revendications précédentes, cette formulation étant sous la forme d'une capsule, d'un comprimé, d'une poudre, d'un granulé ou d'un sachet.

4. Formulation pharmaceutique selon l'une quelconque des revendications précédentes, dans laquelle ledit excipient comprend au moins un ou un mélange de charges, de liants, de désintégrants, de lubrifiants et d'agents de glissement.

5. Formulation pharmaceutique selon l'une quelconque des revendications précédentes, comprenant en outre, comme agent de charge, au moins un ou un mélange de cellulose microcristalline, de pullulane, de dextrine, de mannitol, de sorbitol, de lactose, de phosphate dicalcique, de phosphate de calcium dibasique anhydre, l'agent de charge préféré étant la cellulose monocristalline ou le pullulane, ou un mélange bien proportionné de ceux-ci.

6. Formulation pharmaceutique selon l'une quelconque des revendications précédentes, dans laquelle ledit agent de liaison comprend au moins un ou un mélange bien proportionné de polyvinylpyrrolidone (povidone), d'hydroxypropyl méthylcellulose, d'hydroxypropyl cellulose, d'hydroxyéthyl cellulose, d'amidon prégélatinisé, d'alginate de sodium, de chitosane, de carboxyméthyl cellulose, de méthyl cellulose, d'éthyl cellulose, et d'autres dérivés de cellulose, l'agent de liaison préféré étant la polyvinylpyrrolidone.

7. Formulation pharmaceutique selon l'une quelconque des revendications précédentes, dans laquelle ledit agent de glissement comprend au moins un ou un mélange bien proportionné de dioxyde de silicone colloïdale, de talc, de silicate d'aluminium, de phosphate de calcium tribasique, de silicate de magnésium, l'agent glissant préféré étant le dioxyde de silicone colloïdale.

8. Formulation pharmaceutique selon l'une quelconque des revendications précédentes, dans laquelle ledit désintégrant comprend au moins un ou un mélange bien proportionné de glycolate d'amidon sodique, de croscarmellose sodique, de crospovidone, d'alginate de sodium, de gommes, d'amidon et de silicate de magnésium aluminium, le désintégrant préféré étant le croscarmellose sodique.

9. Formulation pharmaceutique selon l'une quelconque des revendications précédentes, dans laquelle ledit lubrifiant comprend au moins un ou un mélange bien proportionné de fumarate de stéaryle sodique, de stéarate de magnésium, de polyéthylène glycol, d'acide stéarique, de stéarates métalliques, d'acide borique, de benzoate et d'acétate de chlorure de sodium, de sodium ou magnésium lauryle sulfate, le lubrifiant préféré étant le stéarate de magnésium.

10. Procédé pour préparer une formulation pharmaceutique selon l'une quelconque des revendications précédentes, comprenant les étapes de :
a. enrobage de granulés de vildagliptine avec du pullulane ou de l'alcool polyvinylique, ou avec un mélange bien proportionné de ceux-ci,
b. mélange des granulés enrobés avec une charge, un liant et des agents désintégrants,
c. ajout de stéarate de magnésium dans le mélange obtenu, et la poursuite de son mélange,
d. compression du mélange en comprimés, ou son chargement dans des capsules,
e. enrobage de film des comprimés compressés.

11. Formulation pharmaceutique selon l'une quelconque des revendications précédentes, constituée de :
a. un coeur comprenant les ingrédients suivants :
a. de la vildagliptine ou un sel pharmaceutiquement acceptable ou un polymorphe de celle-ci de 3,5 à 90 % en poids,
b. du pullulane de 0,2 à 10 % en poids,
c. de la cellulose microcristalline de 2,0 à 90,0 % en poids,
d. de la polyvinylpyrrolidone de 0,25 à 10,0 % en poids,
e. du croscamellose sodique de 0,2 à 20,0 % en poids,
f. du dioxyde de silicone colloïdale de 0,1 à 5,0 % en poids,
g. du stéarate de magnésium de 0,1 à 5,0 % en poids,
b. un enrobage de granulé comprenant les ingrédients suivants :
a. de l'alcool polyvinylique de 0,5 à 5,0 % en poids ou
b. du pullulane ou un mélange associé de 0,5 à 5,0 % en poids.

12. Formulation pharmaceutique selon l'une quelconque des revendications précédentes pour prévenir ou traiter le diabète sucré chez les mammifères et en particulier chez les humains.
